# FASCICULE DE BREVET EUROPEEN

(11) **EP 3 791 886 B1**
(45) Date de publication et mention de la délivrance du brevet: **21.12.2022**
(21) Numéro de dépôt: 20194266.1
(22) Date de dépôt: 03.09.2020
(51) Int. Cl.: A61K 33/00, A61B 17/22, A61K 9/00, A61M 16/10, A61P 9/10

(54) **ARGON ASSOCIÉ À UNE THROMBECTOMIE EN CAS D'ACCIDENT VASCULAIRE CÉRÉBRAL ISCHÉMIQUE**
ARGON IM ZUSAMMENHANG MIT EINER THROMBEKTOMIE IM FALLE EINES ISCHÄMISCHEN SCHLAGANFALLS
ARGON ASSOCIATED WITH A THROMBECTOMY IN THE EVENT OF ISCHEMIC STROKE

(30) Priorité: 12.09.2019 FR 1910063
(43) Date de publication de la demande: 17.03.2021
(73) Titulaire: L'Air Liquide, société anonyme pour l'Étude et l'Exploitation des procédés Georges Claude, 75007 Paris (FR); Centre National de la Recherche Scientifique, 75016 Paris (FR); Aix-Marseille Université, 13007 Marseille (FR)
(72) Inventeur: FARJOT, Géraldine, 78350 Les Loges en Josas (FR); BILLOET, Catherine, 94250 Gentilly (FR); VELLY, Lionel, 13007 Marseille (FR); BROCHIER, Thomas, 13007 Marseille (FR)
(74) Mandataire: Air Liquide

(56) Documents cités:
- EP-A1- 1 541 156
- EP-A1- 3 424 550
- WO-A1-2008/132239
- A.J. GARDNER ET AL: "Moving to human trials for argon neuroprotection in neurological injury: a narrative review", BRITISH JOURNAL OF ANAESTHESIA., vol. 120, no. 3, 1 mars 2018 (2018-03-01), pages 453-468, XP055693879, GB ISSN: 0007-0912, DOI: 10.1016/j.bja.2017.10.017
- SHUANG MA ET AL: "Argon Inhalation for 24 Hours After Onset of Permanent Focal Cerebral Ischemia in Rats Provides Neuroprotection and Improves Neurologic Outcome :", CRITICAL CARE MEDICINE., vol. 47, no. 8, 1 août 2019 (2019-08-01), pages e693-e699, XP055693877, US ISSN: 0090-3493, DOI: 10.1097/CCM.0000000000003809

## Description

La présente invention concerne un médicament gazeux inhalable contenant un mélange binaire d'argon et d'oxygène, pour une utilisation en association avec une thrombectomie mécanique pour traiter, atténuer ou résorber des lésions cérébrales consécutives à un accident vasculaire cérébral ischémique (AVCi) chez un individu, i.e. un patient, tel que défini dans les revendications.

L'accident vasculaire cérébral ischémique ou AVCi est une cause majeure de morbi-mortalité. De plus, la récupération neurologique et fonctionnelle après un AVCi est souvent incomplète. Ainsi, après 1 an, la moitié des survivants souffrent à divers degrés de pathologies motrices ou mentales. L'AVCi est mondialement la première cause de handicap chez l'adulte et la troisième cause de mortalité.

La majorité des AVCi sont dus à un caillot de sang qui obstrue un vaisseau sanguin dans le cerveau d'une personne, ce qui bloque le flux sanguin, donc l'irrigation et l'oxygénation d'une région cérébrale, conduisant à la fragilisation puis la mort des neurones dans la zone non-irriguée.

Ces dommages se produisent immédiatement pendant l'AVC mais aussi après l'AVC, c'est-à-dire qu'ils s'étendent pendant les heures et les jours suivants malgré la résolution du caillot par thrombectomie mécanique, c'est-à-dire une extraction mécanique du caillot, par des agents thrombolytiques engendrant une dissolution pharmacologique du caillot, ou par ces deux méthodes associées l'une à l'autre.

La thrombectomie mécanique, en permettant un retrait rapide du caillot de sang, a largement amélioré par rapport à la thrombolyse seule, le pronostic post-AVC. Toutefois, moins de la moitié des patients sortent avec un score clinique leur permettant de vivre de façon autonomes 90 jours après l'AVC, et une grande majorité d'entre eux souffrent de séquelles, voire même décèdent dans les suites du traitement.

Tous les traitements actuels de l'AVC ont pour but de restaurer le flux cérébral. Dit autrement, aucun des traitements existants ne traite les cellules du cerveau fragilisées par les conséquences de l'AVC, notamment défaut d'oxygénation, inflammation, œdème, stress oxydatif....

Par ailleurs, il existe des études portant sur les effets protecteurs de l'argon sur différents modèles in vitro et in vivo d'agression des cellules cérébrales. Toutefois, celles qui ont étudié les effets d'une exposition à l'argon dans le cadre d'une ischémie cérébrale, ont été réalisées dans des modèles expérimentaux de rongeurs reproduisant faiblement les conditions humaines de l'AVC ischémique et qui n'ont jamais donné lieu au moindre médicament mis sur le marché.

Ceci s'explique par le fait que ces modèles de rongeurs ont montré leurs limites à reproduire la fonction cérébrale et la pathologie humaine. En effet, les différences structurales et fonctionnelles entre un cerveau humain et un cerveau de rongeur sont trop importantes et, de fait, à ce jour, aucun traitement des cellules fragilisées par une ischémie cérébrale ayant démontré un effet chez les rongeurs n'a vu son effet se reproduire chez l'homme. En d'autres termes, les observations faites chez le rongeur ne sont pas transposables à l'être humain dans cette pathologie, en particulier en ce qui concerne les modalités précises d'utilisation et de posologie d'un nouveau traitement.

EP-A-3424550 divulgue une composition pharmaceutique comprenant un mélange gazeux de xénon et d'argon pour prévenir et / ou traiter les insultes ischémiques, par exemple l'ischémie cérébrale. Au vu de cela, un problème est de pouvoir améliorer le traitement des accidents vasculaires cérébraux ischémiques (AVCi) chez l'être humain de manière à traiter, atténuer ou résorber des lésions cérébrales consécutives à un tel AVCi.

Une solution selon l'invention concerne un médicament gazeux inhalable formé d'un mélange binaire d'argon et d'oxygène gazeux pour une utilisation en association avec une thrombectomie mécanique pour traiter, atténuer ou résorber des lésions cérébrales consécutives à un accident vasculaire cérébral ischémique (AVCi) chez un individu, i.e. un être humain, tel que défini dans les revendications.

Autrement dit, l'invention porte sur un médicament gazeux inhalable formé d'un mélange binaire d'argon et d'oxygène gazeux pour une utilisation dans une méthode de traitement, d'atténuation ou de résorption des lésions cérébrales consécutives à un accident vasculaire cérébral ischémique (AVCi) chez un individu devant subir subséquemment une thrombectomie mécanique, ladite méthode comprenant :
(a) l'administration du médicament gazeux par inhalation après ledit accident vasculaire cérébral ischémique (AVCi), ledit médicament gazeux contenant une proportion volumique d'argon comprise entre 50 et 70%,
(b) suivie par une thrombectomie mécanique avec extraction mécanique d'au moins une partie du caillot sanguin résultant de l'AVCi,
et dans laquelle l'administration du médicament gazeux par inhalation :
i) est commencée le plus tôt possible après un diagnostic d'AVCi, et
ii) est maintenue pendant toute la thrombectomie, et
iii) se poursuit après une reperfusion consécutive à l'extraction mécanique d'au moins une partie du caillot sanguin résultant de l'AVCi.

Le médicament gazeux est donc sous une forme adaptée à une administration par inhalation chez un individu devant subir subséquemment une thrombectomie mécanique en vue d'éliminer tout ou partie du caillot sanguin lié à l'AVCi, l'administration du médicament gazeux commençant après l'accident vasculaire cérébral ischémique (AVCi), en particulier après diagnostic, i.e. confirmation, de l'AVCi, et avant le début de la thrombectomie mécanique et se poursuit pendant la thrombectomie mécanique et aussi après la thrombectomie mécanique, c'est-à-dire après reperfusion. Selon l'invention, le médicament gazeux est formé d'un mélange binaire d'argon et d'oxygène et contient une proportion volumique d'argon comprise entre 50 et 70%.

Selon le mode de réalisation considéré, le médicament gazeux de l'invention peut comprendre l'une ou plusieurs des caractéristiques suivantes :
- la proportion volumique d'argon est comprise entre 50 et 65%.
- l'argon est mélangé avec un gaz contenant au moins 21% en volume d'oxygène, de préférence au moins 30% d'oxygène (% en vol.).
- l'argon gazeux est mélangé avec 35 à 50% d'oxygène.
- il est formé d'un mélange binaire d'argon et d'oxygène, dans lequel la proportion volumique d'argon est égale à 60% et la proportion volumique d'oxygène est égale à 40%.
- il ne contient pas de xénon.
- il est sous une forme adaptée à une administration par inhalation avant, pendant et après la thrombectomie mécanique.
- il est sous une forme adaptée à une administration inhalée par voie nasale, buccale ou pharyngée, y compris naso-buccale.

Selon l'invention, le médicament gazeux est sous une forme adaptée à une administration par inhalation le plus tôt possible après un diagnostic d'AVCi, c'est-à-dire dans la ou les minutes qui suivent le diagnostic d'AVCi de manière à éviter une aggravation de l'AVCi. Plus l'administration se fait rapidement, c'est-à-dire sans tarder, plus les effets délétères de l'AVCi chez le patient peuvent être réduits, i.e. limités ou minimisés.

Selon l'invention, le médicament gazeux est sous une forme adaptée à une administration par inhalation après la thrombectomie mécanique, c'est-à-dire qu'elle est maintenue pendant plusieurs minutes ou dizaines de minutes après la reperfusion subséquente à la thrombectomie, de préférence pendant au moins 10 minutes, de préférence encore pendant au moins 15 à 20 minutes, avantageusement pendant au moins 30 minutes, et même jusqu'à au moins 60 minutes, voire plus longtemps, après reperfusion.

Selon le mode de réalisation considéré, le médicament gazeux de l'invention peut comprendre l'une ou plusieurs des caractéristiques suivantes :
- l'individu est un adulte, un adolescent ou un enfant.
- l'individu est un adulte d'au moins 40 ans, de préférence d'au moins 50 ans.
- il est associé à une thrombectomie mécanique avec ou sans thrombolyse médicamenteuse, c'est-à-dire avec éventuellement administration d'un agent médicamenteux permettant de fluidifier le sang et de désagréger au moins une partie du caillot.
- la proportion d'argon est choisie pour agir efficacement sur les cellules cérébrales de la zone de pénombre fragilisées par l'AVCi.
- la proportion d'argon est choisie pour améliorer la plasticité neuronale des cellules cérébrales de la zone de pénombre.
- les lésions cérébrales sont de types inflammations, rupture de barrière hémato-encéphalique, apoptose cellulaire, nécrose tissulaire, œdème ou autre.
- la thrombectomie mécanique comprend une extraction mécanique du caillot sanguin au moyen d'une sonde insérée dans un vaisseau sanguin, en particulier une artère.
- la méthode de traitement, d'atténuation ou de résorption des lésions cérébrales consécutives à l'AVCi comprend une sédation du patient au moins pendant la thrombectomie mécanique, c'est-à-dire que la thrombectomie mécanique est opérée sur un patient sous anesthésie générale ou anesthésie locale, de préférence combinée à une sédation consciente.
- la méthode de traitement, d'atténuation ou de résorption des lésions cérébrales consécutives à l'AVCi comprend une sédation du patient par administration d'un ou plusieurs produits sédatifs, analgésiques ou anesthésiques, par exemple halothane, isoflurane, desflurane, sevoflurane, thiopental, propofol, fentanyl, midazolam, propofol, remifentanyl ou autre.
- le médicament gazeux est conditionné dans un récipient de gaz sous pression, en particulier une bouteille de gaz sous pression.
- le gaz provenant du récipient de gaz sous pression est administré au patient via une inhalation par voie nasale, buccale ou pharyngée, i.e. trachéale, y compris naso-buccale, au moyen d'une interface respiratoire adaptée, tel qu'un masque, des canules nasales ou une sonde trachéale par exemple.
- le médicament gazeux provenant du récipient de gaz sous pression est administré au patient au moyen d'un dispositif médical d'administration de gaz, c'est-à-dire soit une valve adaptée à la respiration spontanée du médicament gazeux, soit un appareil de ventilation assistée (ou ventilateur médical) alimentant le patient en médicament gazeux, de préférence via une interface respiratoire adaptée reliée fluidiquement au dispositif médical d'administration de gaz par l'intermédiaire d'un tuyau flexible ou analogue.

La méthode d'administration de l'argon chez un individu, i.e. un patient, ayant un AVCi est par exemple la suivante :
a) on fait inhaler un mélange 60% Ar/40% O₂ (% en vol.) au moyen d'un dispositif respiratoire adapté à une administration nasale, buccale ou trachéale, par exemple un masque respiratoire ou une sonde trachéale, à un patient immédiatement après confirmation du diagnostic d'AVC par imagerie cérébrale, par exemple de type Scanner (TDM) ou IRM ;
b) on poursuit l'administration par inhalation du mélange Ar/O₂ pendant toute la préparation d'une procédure de thrombectomie mécanique, typiquement pendant 15 à 45 minutes environ, et pendant la procédure de thrombectomie mécanique proprement-dite, comme décrit ci-avant ; et
c) de préférence, on arrête l'administration par inhalation du mélange Ar/O₂ après la procédure de thrombectomie mécanique, c'est-à-dire après le retrait du caillot au moyen d'une sonde, plusieurs minutes après la thrombectomie, préférentiellement au moins 10 à 15 minutes après reperfusion, de préférence encore entre 10 et 60 minutes après reperfusion, typiquement environ 30 minutes après reperfusion.

De préférence, la procédure de thrombectomie mécanique s'accompagne de, i.e. est associée à, une thrombolyse médicamenteuse avec administration d'un agent médicamenteux permettant de fluidifier le sang et de désagréger le caillot.

Par ailleurs, la procédure de thrombectomie mécanique est effectuée avec anesthésie générale ou locale (e.g. locorégionale) ou sédation consciente du patient.

L'invention va maintenant être mieux comprise grâce à la description détaillée suivante issue d'une étude chez le singe macaque rhésus, faite à titre illustratif mais non limitatif, en référence aux figures annexées parmi lesquelles :
[Fig. 1] schématise des volumes infarci (gris foncé) par rapport aux volumes de substance blanche (gris clair), issu d'images IRM en vues sagittales, coronales et axiales des cerveaux d'un animal représentatif du « groupe argon » et d'un animal représentatif du « groupe contrôle » ; et
[Fig. 2] représente la distribution des volumes de lésion dans le « groupe contrôle » et le « groupe argon ».

Contrairement aux modèles rongeurs, les primates non-humains ont une structure cérébrale gyrencéphale, une architecture vasculaire et micro-vasculaire, des systèmes de régulation homéostatique et de coagulation très proches de ceux de l'être humain. De ce fait, dans le cadre de la présente invention, on a utilisé un modèle primate puisqu'il constitue un modèle idéal d'exploration précise des conditions cliniques du traitement de l'AVC ischémique (AVCi).

Un tel modèle primate n'a jamais été utilisé auparavant pour étudier le traitement des AVCi, c'est-à-dire pour tenter de trouver un moyen efficace pour traiter, atténuer ou résorber tout ou partie des lésions cérébrales consécutives à un AVCi chez l'être humain.

Les primates utilisés dans l'étude sont des macaque rhésus, chez lesquels il a été possible d'obtenir un modèle stable et reproductible d'ischémie réversible mimant un AVCi et sa reperfusion par thrombectomie mécanique, comme expliqué ci-après.

Après approbation du projet par comité d'éthique en expérimentation animale, 6 macaques rhésus ont été randomisés en 2 groupes de 3 animaux par groupe, à savoir :
- un groupe traité par argon ou « groupe argon » inhalant, pendant 90 min au total, un mélange gazeux binaire formé de 60% argon et 40% oxygène (% vol.), l'inhalation du gaz débutant 30 min après le début de l'ischémie et se prolongeant pendant et après l'acte de thrombectomie (avec ou sans thrombolyse).
- un « groupe contrôle » inhalant un mélange gazeux formé de 60% azote et 40% oxygène (% vol.).

Ces groupes sont soumis à un modèle d'ischémie cérébrale focale transitoire sans craniectomie consistant en une occlusion sous anesthésie de l'artère cérébrale moyenne (ACM) par le déploiement jusqu'à l'arrêt du flux artériel d'un micro-ressort rigide, appelé « micro-coïl », dans le segment M2 de l'ACM droite.

Après 90 min d'ischémie, qui est le temps moyen usuel entre les premiers signes d'un AVCi et la reperfusion cérébrale, le « micro-coïl » a été retiré et la revascularisation confirmée par une artériographie. Cette procédure mime un caillot sanguin et son retrait par thrombectomie mécanique.

Tous les animaux sont suivis en IRM haute résolution (Prisma 3 Tesla, Siemens) plusieurs jours avant et directement après l'ischémie (3D-T1, 3D-T2, 3D-FLAIR, DTI, TOF) afin de quantifier le volume cérébral ischémié (i.e. restriction du coefficient apparent de diffusion) et l'œdème cérébral (i.e. T2-FLAIR).

Par ailleurs, la récupération fonctionnelle a été suivie en phase aigüe chez 1 animal contrôle et sur le long terme chez deux animaux du groupe argon c'est-à-dire sur 3 mois post-ischémie. Les évaluations ont été réalisées sur le plan de la récupération neurologique clinique et fonctionnelle avec des tests comportementaux évaluant leurs performances sensori-motrices fines.

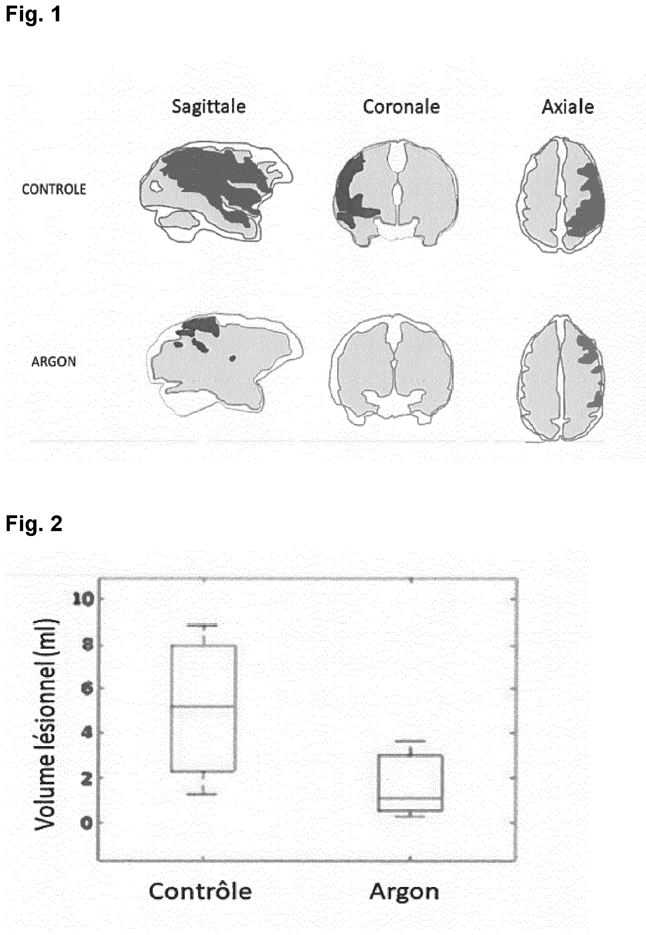

Les résultats obtenus, visibles sur [Fig. 1] et [Fig. 2], montrent que l'ischémie cérébrale pratiquée sur le modèle primate a provoqué un volumineux infarctus cérébral de 5.4 ±3.3 cm³, englobant l'ensemble du lobe pariétal et s'étendant aux lobes temporal et frontal des primates du " groupe contrôle ".

Dans le « groupe argon », l'exposition à l'argon inhalé a entrainé une diminution significative du volume cérébral infarci (i.e. réduction de 83%; P<0,05) et du volume d'œdème cérébral (i.e. réduction de 85%; P<0,05) par rapport au « groupe contrôle ». Ainsi, le volume lésionnel est de l'ordre de 5 ml dans « groupe contrôle » mais seulement de l'ordre de 1 ml dans le « groupe argon ».

Sur les images IRM, il a aussi été observé une rupture de la barrière hématoencéphalique chez les animaux du "groupe contrôle", qui est un phénomène connu lors de l'AVC chez l'humain, alors que la barrière hématoencéphalique a été préservée chez les animaux du "groupe argon".

En d'autres termes, l'argon préserve la barrière hématoencéphalique pendant l'AVC et protège donc le cerveau des atteintes que cette rupture pourrait provoquer.

Au plan comportemental, les animaux du « groupe argon » traités par inhalation d'argon et suivis à long terme (i.e. 3 mois) ont présenté une récupération rapide avec seulement un déficit neurologique mineur et transitoire en post-ischémie, à savoir une parésie de la patte gauche. Leurs performances aux tests comportementaux n'ont pas été significativement altérées en post-ischémie par rapport aux données de bases.

Chez l'animal du « groupe contrôle », la récupération a été lente, voire inexistante, avec persistance de déficits neurologiques plus ou moins importants.

Ces résultats montrent que l'argon inhalé permet de traiter les cellules cérébrales fragilisées lors d'une ischémie post-AVC et donc de réduire l'étendue et le volume final des lésions cérébrales d'un tel AVC, après reperfusion par une thrombectomie mécanique, c'est-à-dire administré pendant et après une procédure de résolution de l'AVCi par thrombectomie mécanique, avec ou sans thrombolyse, visant à éliminer le caillot sanguin qui s'est formé et qui a provoqué l'AVC.

Plus précisément, l'argon permet une amélioration de la plasticité neuronale dans la zone de pénombre, c'est-à-dire la zone des cellules fragilisées par l'ischémie cérébrale, lorsque l'événement AVCi est résolu en quelques heures par une thrombectomie mécanique avec ou sans thrombolyse.

Dit autrement, l'argon traite le déficit neurologique et permet par ailleurs une récupération accélérée, donc un retour plus rapide à la vie normale.

Ces résultats obtenus en modèle primate peuvent être extrapolés chez l'être humain, contrairement à ceux obtenus chez les rongeurs.

Compte tenu de ces observations, il est préférable de débuter l'administration d'argon par inhalation le plus tôt possible après le diagnostic et pendant toute la procédure de résolution de l'AVC, c'est-à-dire pendant la thrombectomie mécanique, avec ou sans thrombolyse, ainsi que pendant au moins 30 minutes après la reperfusion suivant l'élimination du caillot sanguin.

L'argon est en mélange avec de l'oxygène ou co-administré avec de l'oxygène. Avantageusement, on met en œuvre un mélange Ar/O₂ contenant environ 60% d'argon et 40% d'oxygène, étant donné que cette dose a été démontrée comme efficace tout en permettant de maintenir une oxygénation compatible avec les besoins vitaux de l'individu traité.

## Revendications

1. Médicament gazeux inhalable formé d'un mélange binaire d'argon et d'oxygène gazeux pour une utilisation dans une méthode de traitement, d'atténuation ou de résorption des lésions cérébrales consécutives à un accident vasculaire cérébral ischémique (AVCi) chez un individu devant subir subséquemment une thrombectomie mécanique, ladite méthode comprenant :
(a) l'administration du médicament gazeux par inhalation après ledit accident vasculaire cérébral ischémique (AVCi), ledit médicament gazeux contenant une proportion volumique d'argon comprise entre 50 et 70%,
(b) suivie par une thrombectomie mécanique avec extraction mécanique d'au moins une partie du caillot sanguin résultant de l'AVCi,
et dans laquelle l'administration du médicament gazeux par inhalation :
i) est commencée le plus tôt possible après un diagnostic d'AVCi,
ii) est maintenue pendant toute la thrombectomie, et
iii) se poursuit après une reperfusion consécutive à l'extraction mécanique d'au moins une partie du caillot sanguin résultant de l'AVCi.

2. Médicament pour utilisation selon la revendication précédente, **caractérisé en ce que** la proportion volumique d'argon est comprise entre 50 et 65%.

3. Médicament pour utilisation selon la revendication 1, **caractérisé en ce que** l'argon gazeux est mélangé avec de 30 à 50% d'oxygène.

4. Médicament pour utilisation selon l'une des revendications précédentes, **caractérisé en ce que** la proportion volumique d'argon est égale à 60% et la proportion volumique d'oxygène est égale à 40%.

5. Médicament pour utilisation selon l'une des revendications précédentes, **caractérisé en ce qu'**il est sous une forme adaptée à une administration inhalée par voie nasale, buccale ou pharyngée.

6. Médicament pour utilisation selon la revendication 1, **caractérisé en ce que** l'administration du médicament gazeux par inhalation se poursuit plusieurs minutes après reperfusion.

7. Médicament pour utilisation selon la revendication 6, **caractérisé en ce que** l'administration du médicament gazeux par inhalation se poursuit au moins 10 minutes après reperfusion.

8. Médicament pour utilisation selon l'une des revendications 6 ou 7, **caractérisé en ce que** l'administration du médicament gazeux par inhalation se poursuit au moins 30 minutes après reperfusion.

9. Médicament pour utilisation selon l'une des revendications 1 à 4, **caractérisé en ce que** l'argon est conditionné dans un récipient de gaz sous pression.

10. Médicament pour utilisation selon l'une des revendications précédentes, **caractérisé en ce que** l'individu est un adulte, un adolescent ou un enfant.

11. Médicament pour utilisation selon la revendication 1, **caractérisé en ce que** la thrombectomie mécanique comprend une extraction mécanique de tout le caillot sanguin résultant de l'AVCi.

12. Médicament pour utilisation selon l'une des revendications 1 ou 11, **caractérisé en ce que** la thrombectomie mécanique est associée à une thrombolyse médicamenteuse.

13. Médicament pour utilisation selon la revendication 1, **caractérisé en ce que** la méthode comprend une confirmation du diagnostic d'AVCi par imagerie cérébrale.

14. Médicament pour utilisation selon la revendication 1, **caractérisé en ce que** la méthode comprend une anesthésie du patient qu'elle soit générale ou locale, de préférence avec sédation consciente.

## Patentansprüche

1. Inhalierbares gasförmiges Arzneimittel, das aus einem binären Gemisch aus Argon- und Sauerstoffgas gebildet ist, zur Verwendung in einem Verfahren zur Behandlung, Linderung oder Resorption von Hirnschäden im Anschluss an einen ischämischen Schlaganfall (iCVA, Hirninfarkt) bei einem Individuum, das anschließend einer mechanischen Thrombektomie unterzogen wird, wobei das Verfahren Folgendes umfasst:
(a) Verabreichung des gasförmigen Arzneimittels durch Inhalation nach dem ischämischen Schlaganfall (iCVA), wobei das gasförmige Arzneimittel einen Volumenanteil von Argon zwischen 50 und 70 % enthält,
(b) gefolgt von einer mechanischen Thrombektomie mit mechanischer Entfernung mindestens eines Teils des aus dem iCVA resultierenden Blutgerinnsels,
und wobei die Verabreichung des gasförmigen Arzneimittels durch Inhalation :
i) so bald wie möglich nach einer iCVA-Diagnose begonnen wird,
ii) während der gesamten Thrombektomie aufrechterhalten wird, und
iii) nach einer Reperfusion im Anschluss an die mechanische Entfernung mindestens eines Teils des aus dem iCVA resultierenden Blutgerinnsels fortgesetzt wird.

2. Arzneimittel zur Verwendung nach dem vorhergehenden Anspruch, **dadurch gekennzeichnet, dass** der Volumenanteil von Argon zwischen 50 und 65 % liegt.

3. Arzneimittel zur Verwendung nach Anspruch 1, **dadurch gekennzeichnet, dass** das Argongas mit 30 bis 50 % Sauerstoff vermischt ist.

4. Arzneimittel zur Verwendung nach einem der vorhergehenden Ansprüche, **dadurch gekennzeichnet, dass** der Volumenanteil von Argon gleich 60 % und der Volumenanteil von Sauerstoff gleich 40 % ist.

5. Arzneimittel zur Verwendung nach einem der vorhergehenden Ansprüche, **dadurch gekennzeichnet, dass** es in einer zur inhalativen Verabreichung über den nasalen, bukkalen oder pharyngealen Weg geeigneten Form vorliegt.

6. Arzneimittel zur Verwendung nach Anspruch 1, **dadurch gekennzeichnet, dass** die Verabreichung des gasförmigen Arzneimittels durch Inhalation mehrere Minuten lang nach der Reperfusion fortgesetzt wird.

7. Arzneimittel zur Verwendung nach Anspruch 6, **dadurch gekennzeichnet, dass** die Verabreichung des gasförmigen Arzneimittels durch Inhalation mindestens 10 Minuten lang nach der Reperfusion fortgesetzt wird.

8. Arzneimittel zur Verwendung nach einem der Ansprüche 6 oder 7, **dadurch gekennzeichnet, dass** die Verabreichung des gasförmigen Arzneimittels durch Inhalation mindestens 30 Minuten lang nach der Reperfusion fortgesetzt wird.

9. Arzneimittel zur Verwendung nach einem der Ansprüche 1 bis 4, **dadurch gekennzeichnet, dass** das Argon in einem Druckgasbehälter verpackt ist.

10. Arzneimittel zur Verwendung nach einem der vorhergehenden Ansprüche, **dadurch gekennzeichnet, dass** das Individuum ein Erwachsener, ein Jugendlicher oder ein Kind ist.

11. Arzneimittel zur Verwendung nach Anspruch 1, **dadurch gekennzeichnet, dass** die mechanische Thrombektomie eine mechanische Entfernung des gesamten aus dem iCVA resultierenden Blutgerinnsels umfasst.

12. Arzneimittel zur Verwendung nach einem der Ansprüche 1 oder 11, **dadurch gekennzeichnet, dass** die mechanische Thrombektomie im Zusammenhang mit einer medikamentösen Thrombolyse erfolgt.

13. Arzneimittel zur Verwendung nach Anspruch 1, **dadurch gekennzeichnet, dass** das Verfahren eine Bestätigung der Diagnose von iCVA durch zerebrale Bildgebung umfasst.

14. Arzneimittel zur Verwendung nach Anspruch 1, **dadurch gekennzeichnet, dass** das Verfahren eine Allgemein- oder Lokalanästhesie des Patienten, vorzugsweise mit Sedierung bei Bewusstsein, umfasst.

## Claims

1. Inhalable gaseous medicament made up of a binary mixture of argon gas and oxygen gas for use in a method for treating, reducing or resorbing brain lesions subsequent to an ischemic stroke in an individual who must subsequently undergo a mechanical thrombectomy, said method comprising:
(a) the administration of the gaseous medicament by inhalation after said ischemic stroke, said gaseous medicament containing a proportion by volume of argon of between 50 and 70%,
(b) followed by a mechanical thrombectomy with mechanical extraction of at least one portion of the blood clot resulting from the ischemic stroke,
and in which the administration of the gaseous medicament by inhalation:
(i) is begun as early as possible after an ischemic stroke diagnosis,
(ii) is maintained throughout the thrombectomy, and
(iii) is continued after a reperfusion subsequent to the mechanical extraction of at least one portion of the blood clot resulting from the ischemic stroke.

2. Medicament for use according to the preceding claim, **characterized in that** the proportion by volume of argon is between 50 and 65%.

3. Medicament for use according to Claim 1, **characterized in that** the argon gas is mixed with 30 to 50% of oxygen.

4. Medicament for use according to one of the preceding claims, **characterized in that** the proportion by volume of argon is equal to 60% and the proportion by volume of oxygen is equal to 40%.

5. Medicament for use according to one of the preceding claims, **characterized in that** it is in a form suitable for inhaled administration by the nasal, buccal or pharyngeal route.

6. Medicament for use according to Claim 1, **characterized in that** the administration of the gaseous medicament by inhalation is continued for several minutes after reperfusion.

7. Medicament for use according to Claim 6, **characterized in that** the administration of the gaseous medicament by inhalation is continued for at least 10 minutes after reperfusion.

8. Medicament for use according to either of Claims 6 and 7, **characterized in that** the administration of the gaseous medicament by inhalation is continued for at least 30 minutes after reperfusion.

9. Medicament for use according to one of Claims 1 to 4, **characterized in that** the argon is packaged in a pressurized gas container.

10. Medicament for use according to one of the preceding claims, **characterized in that** the individual is an adult, an adolescent or a child.

11. Medicament for use according to Claim 1, **characterized in that** the mechanical thrombectomy comprises a mechanical extraction of the entire blood clot resulting from the ischemic stroke.

12. Medicament for use according to either of Claims 1 and 11, **characterized in that** the mechanical thrombectomy is combined with a drug-based thrombolysis.

13. Medicament for use according to Claim 1, **characterized in that** the method comprises a confirmation of the ischemic stroke diagnosis by brain imaging.

14. Medicament for use according to Claim 1, **characterized in that** the method comprises anaesthesia of the patient, whether general or local, preferably with conscious sedation.
